# EUROPÄISCHE PATENTSCHRIFT

(11) **EP 2 160 134 B1**
(45) Veröffentlichungstag und Bekanntmachung des Hinweises auf die Patenterteilung: **14.11.2012**
(21) Anmeldenummer: 08760153.0
(22) Anmeldetag: 28.05.2008
(51) Int. Cl.: A61B 6/00, A61B 6/03, H01P 1/06

(54) **KONTAKTLOSER DREHÜBERTRAGER**
CONTACTLESS ROTARY TRANSMITTER
TRANSMETTEUR ROTATIF SANS CONTACT

(30) Priorität: 21.06.2007 DE 102007029109
(43) Veröffentlichungstag der Anmeldung: 10.03.2010
(73) Patentinhaber: Schleifring und Apparatebau GmbH, 82256 Fürstenfeldbruck (DE)
(72) Erfinder: LINDORFER, Stephan, 81245 München (DE)
(74) Vertreter: Lohr, Georg
(86) Internationale Anmeldenummer: PCT/EP2008/056558
(87) Internationale Veröffentlichungsnummer: WO 2008/155200

(56) Entgegenhaltungen:
- EP-A- 0 398 581
- EP-A- 0 982 681
- EP-A- 1 351 194
- EP-A- 1 517 461
- WO-A-2004/032364
- DE-A1- 10 241 583
- US-A- 5 208 581
- US-A1- 2005 012 646
- US-B1- 6 362 757

## Beschreibung

### Technisches Gebiet

Die Erfindung betrifft ein Datenübertragungssystem zur Übertragung von Daten zwischen einem rotierenden Teil und einem stationären Teil, insbesondere zwischen dem rotierenden Teil und dem stationären Teil eines Computertomographen sowie einen Computertomographen mit einem entsprechenden Übertragungssystem.

### Stand der Technik

Bei drehbaren Einheiten wie Radaranlagen oder auch Computertomographen und auch bei linear beweglichen Einheiten wie Kran- und Förderanlagen ist es notwendig, zwischen gegeneinander beweglichen Einheiten elektrische Signale bzw. Energie zu übertragen. Hierzu sind meist eine Leiterstruktur in der ersten Einheit und ein entsprechender Abgriff in der zweiten Einheit vorgesehen. In den folgenden Ausführungen bezieht sich der Begriff Leiterstrukturen auf alle denkbaren Formen von Leiterstrukturen, welche geeignet sind, elektrische Signale zu führen. Dies bezieht sich auch auf die bekannten kontaktierenden Schleifbahnen bzw. Schleifringe. Wesentlich bei der Übertragung mittels Drehübertragern bzw. linearer "Schleifleitungen", welche auch kontaktlos ausgeführt sein können, ist die geringe Distanz der Übertragung zwischen den gegeneinander beweglichen Einheiten. So kann das Signal wahlweise galvanisch oder berührungslos im Nahfeld der Leiterstrukturen ausgekoppelt werden.

Aus der US 6,433,631 B2 ist eine Vorrichtung zur Datenübertragung in Computertomographen bekannt. Eine Streifenleitung im rotierenden Teil wird mit dem Sendesignal beaufschlagt. Am stationären Teil ist ein Abgriff vorgesehen, welcher in einem geringen Abstand in einer Größenordnung von ca. 1 mm von der Streifenleitung geführt wird. Bei Computertomographen liegt die Länge der Streifenleitung in einer Größenordnung von ca. 5 Metern. Es müssen also bei Datenübertragungsraten von einigen 10 Gigabit pro Sekunde Signale mit einer Bandbreite von mehreren Gigahertz über ein ca. 5 Meter langes Leitungssystem geleitet werden. Es treten hierbei insbesondere bei höheren Frequenzen erhebliche dielektrische Verluste auf. Die dielektrischen Verluste werden durch das Material des Dielektrikums der Streifenleitung bestimmt. Materialien mit wenigen dielektrischen Verlusten sind extrem teuer und meist auch mechanisch schwer zu bearbeiten.

In der US 3,457,510 ist ein Duobinär-Kodierer offenbart. Dieser besteht aus einer digitalen Logik und einem anschließenden Bandpassfilter.

In der EP 1 012 899 A ist ein kapazitiver Drehübertrager mit Bandpass-Charakteristik offenbart. Es handelt sich hierbei um ein Bandpassfilter hoher Ordnung mit extrem steilen Filterflanken.

In der WO2006/043268 A1 ist ein Duobinär-Precoder offenbart.

In der WO 2004/032364 A ist ein Verfahren zur Übertragung von Daten zwischen dem rotierenden Teil und dem stationären Teil eines Computertomographen mit einem Drehübertrager zur Übertragung elektrischer Signale offenbart. Weiterhin ist zwischen der Datenquelle und dem Sender eine Kodiereinrichtung vorgesehen, die die digitale Kodierung des Datenstroms derart umsetzt, dass in bestimmten spektralen Bereichen die Leistung wahlweise erhöht oder abgesenkt wird.

In der EP 1 517 461 ist eine optischer, duobinär kodierte Signalübertragung dargestellt, die einen besonders einfachen Mach-Zehnder-Modulator offenbart.

### Darstellung der Erfindung

Aufgabe der Erfindung ist es, einen Computertomographen mit einem Drehübertragungssystem, sowie ein Drehübertragungssystem beispielsweise für Computertomographen vorzustellen, bei dem preisgünstige dielektrische Materialien mit höheren Dämpfungen bei höheren Frequenzen für die Fernsehleiterstruktur (Streifenleitung) eingesetzt werden können.

Eine erfindungsgemäße Lösung dieser Aufgabe ist in den unabhängigen Patentansprüchen angegeben. Weiterbildungen der Erfindung sind Gegenstand der abhängigen Ansprüche.

Ein erfindungsgemäßer Computertomograph ist hier beispielhaft dargestellt. Es werden zwischen dem rotierenden Teil 1 und dem stationären Teil 2 eines Computertomographen Daten übertragen. Es ist wenigstens eine Datenquelle 4 am rotierenden Teil und wenigstens eine Datensenke 5 am stationären Teil vorgesehen. Eine Datenquelle kann beispielsweise ein Röntgendetektor 103 bzw. dessen DAS (Data Acquisition System), oder auch eine beliebige andere Steuereinrichtung oder ein Rechner sein. Es können auch die Daten mehrerer Datenquellen zur Übertragung miteinander kombiniert werden. Eine Datensenke kann ein Rechner 106 zur Auswertung und Aufbereitung der Daten, aber auch eine andere Steuereinheit sein.

Der Drehübertrager weist als wichtigste Bestandteile im rotierenden Teil wenigstens eine erste Sendeeinrichtung 8 sowie eine von dieser gespeiste Sendeleiteranordnung 6 auf. Eine solche erste Sendeeinrichtung empfängt Daten von der Datenquelle und setzt diese zur Übertragung durch die Sendeleiteranordnung um. Die Sendeleiteranordnung umfasst wenigstens einen Leiter zur Führung elektromagnetischer Wellen, der vorzugsweise entlang wenigstens eines Kreissegments oder einer Kreisbahn am rotierenden Teil angebracht ist. Die Sendeleiteranordnung kann beispielsweise mechanische Schleifringe, kontaktlose elektrische Koppelelemente wie induktive oder kapazitive Koppelemente. Ebenso kann die Sendeleiteranordnung eine Kombination aus mehreren unterschiedlichen Koppelelementen umfassen.

Weiterhin umfasst der Drehübertrager im stationären Teil wenigstens eine erste Empfangseinrichtung 9, sowie eine diese speisende Empfangskoppleranordnung 7.

Die Koppler sind passend zur Sendeleiteranordnung ausgebildet. So können beispielsweise kapazitive Koppelflächen in Verbindung mit einer Streifenleitungsstruktur als Sendeleiteranordnung eingesetzt werden. Ebenso können auch optische Prismenkoppler in Verbindung mit einem Lichtwellenleiter, beispielsweise wie einem Spiegelgraben als Sendeleiteranordnung kombiniert werden.

Die Empfangseinrichtung setzt die von der Empfangskoppleranordnung 7 aus der Sendeleiteranordnung 6 empfangenen Signale zur Weiterleitung an die Datensenke um.

Die Empfangseinrichtung 9 und / oder die Datensenke 5 weist eine PLL 10 auf, welche den internen Empfangstakt auf den empfangenen Datenstrom synchronisiert. Weiterhin ist eine Steuereinheit 11 vorgesehen, welche die zumindest eine Datenquelle 4 und/oder die wenigstens eine Sendeeinrichtung 8 ansteuert. Die Steuereinheit kann an jedem der gegeneinander drehbaren Teile, oder auch auf beide Teile aufgeteilt sein. Es wird durch die Steuereinheit eine Empfangsstörung der Empfangseinrichtung und/oder ein Ausrasten der PLL signalisiert und die Aussendung eines speziellen Resynchronisationssignals ausgelöst. Das Resynchronisationssignal kann hierbei wahlweise von der Datenquelle erzeugt werden, die zur Resynchronisation besonders geeignete Daten aussendet. Ebenso kann aber auch die Sendeeinrichtung das Resynchronisationssignal unabhängig von den von der Datenquelle gelieferten Daten erzeugen. Selbst wenn anstelle der von der Datenquelle gelieferten Nutzdaten ein Synchronisationssignal erzeugt wird, führt dies ist zu keinem zusätzlichen Datenverlust, da bei nicht synchronisierter PLL ohnehin keine Daten übertragen werden können.

Die hier beschriebene besonders günstige Ausführungsform eines Computertomographen ist sinngemäß auch bei anderen Anwendungen zur Übertragung von Signalen einer Datenquelle zu einer gegenüber dieser drehbar bzw. linear beweglichen Datensenke anwendbar. Einsatzbeispiele sind allgemeine Drehübertrager, wie sie in Radaranlagen, Rundtaktanlagen oder Kränen eingesetzt werden. Auch können entsprechend der Erfindung Daten über Schleifringe oder Schleifleitungen übertragen werden.

Entsprechend der Erfindung umfasst nun die Sendeeinrichtung 8 einen Kodierer 20, einen Filter 21 sowie einen Treiber 22. Die Signale der Datenquelle 4 in duobinär kodierte Signale zur Übertragung über den Drehübertrager umgesetzt. Ein geeigneter Duobinärkodierer ist beispielsweise in der US 3,457,510 offenbart. Es können durch diese Duobinärkodierung Signale mit niedrigerer Bandbreite gegenüber einer NRZ-Codierung übertragen werden. Ein Duobinärcode ist ein Code aus der Klasse der Correlated Codes. Die Duobinärkodierung erfolgt in einem ersten Schritt durch eine digitale Kodierstufe und in einem zweiten Schritt durch Bandpassfilterung der kodierten digitalen Signale. Eine solche Kodierstufe kann auch ein Precoder sein. Der erste Schritt wird durch den digitalen Kodierer 20 realisiert. Weiterhin erfolgt mittels des Filters 21 die notwendige Filterung der Signale in Verbindung mit der Sendeleiteranordnung 6. Bekannte Sendeleiteranordnung haben typischerweise Bandpasscharakteristik. Ein Beispiel ist in der EP 1 012 899 A offenbart. Das für die Duobinärkodierung notwendige Bandpassfilter wird nun aus dem Filter 21 und der Sendeleiteranordnung 6 gebildet. Vorzugsweise weist dieses Bandpassfilter eine minimale Durchgangsdämpfung bei einem Viertel der Bitrate der digitalen Daten auf. Eine hohe Durchgangsdämpfung sollte bei der Frequenz null und bei einer Frequenz entsprechend der halben Bitrate der digitalen Daten vorgesehen sein. Das Ausgangssignal des Filters 21 wird über den Treiber 22 an die Sendeleiteranordnung 6 übertragen. Die Aufgabe des Treibers 22 ist es, ein Signal mit hinreichend großer Amplitude für eine Last entsprechend der Sendeleiteranordnung 6 zur Verfügung zu stellen. Der Treiber ist hier vorzugsweise ein linearer Verstärker. Es ist auch eine Anordnung mit einem digitalen Treiber nach der Kodierstufe sowie ein dem Treiber nachgeschaltetem Filter realisierbar. Üblicherweise weisen die Sendeleiteranordnungen relativ niedrige Impedanzen im Bereich von einigen Ohm, typischerweise in einem Bereich von 10 bis 100 Ohm, auf. Eine entsprechende Anpassung erfolgt hier durch den Treiber 22. Weiterhin ist es bevorzugter Weise eine Aufgabe des Treibers 22, symmetrische Signale zur Speisung einer differentiellen Sendeleiteranordnung zur Verfügung zu stellen. Beim Einsatz einer differentiellen Sendeleiteranordnung kann eine relativ geringe Abstrahlung hochfrequenter Signale in dem freien Raum erreicht werden. Hierbei können günstige EMV-Eigenschaften erzielt werden. Voraussetzung hierfür ist allerdings ein hochsymmetrisches differentielles Signal. Um dieses zu erzeugen, weist der Treiber vorzugsweise noch Mittel zur Symmetrierung des Signals, wie beispielsweise Symmetrierübertrager oder Baluns auf. Ebenso kann auch ein Precoder, wie in der WO 2006/043268 offenbart, eingesetzt werden.

Eine erfindungsgemäße Empfangseinrichtung 9 umfasst wenigstens einen Verstärker 23 und einen Empfänger 24. Der Verstärker 23 verstärkt die Signale der Empfangskoppleranordnung 7. Da die Empfangskoppleranordnung 7 typischerweise sehr kleine Koppelflächen hat, ist die Koppelkapazität zur Sendeleiteranordnung 6 relativ gering. Entsprechend ist die übertragene Leistung klein. Diese muss nun durch einen entsprechenden Vorverstärker verstärkt werden. Weiterhin ist der Verstärker 23 vorteilhafterweise mit einer Vorrichtung zur Erhöhung der Gleichtaktunterdrückung versehen. Dies kann beispielsweise ein Symmetrierübertrager oder ein Balun sein. Somit werden nur Gegentaktsignale, die den gezielt zu übertragenden Daten von der Sendeleiteranordnung 6 entsprechen, weiterverarbeitet. Störungen, die von außen in die Anordnung eingekoppelt werden, sind vorzugsweise Gleichtaktsignale, welche dann ausgefiltert werden. Der Empfänger 24 wertet die Signale des Verstärkers 23 aus und erzeugt einen Datenstrom, der von der Datensenke 5 weiter verarbeitet werden kann. Hierzu weist der Empfänger 24 einen Duobinärkonverter auf, der die Duobinärsignale dekodiert. Vorzugsweise enthält der Empfänger ein betragsbildendes Element, welches wahlweise den Betrag der Signalspannung oder der Signalleistung bildet. Weiterhin kann der Empfänger 24 eine Filteranordnung zur Kompensation des Frequenzgangs der gesamten Anordnung, insbesondere der Sendeleiteranordnung 6 aufweisen. Hier werden vorzugsweise auch höhere Frequenzanteile, die in der Sendeleiteranordnung 6 stark gedämpft werden, angehoben. Dies ist insbesondere dann sinnvoll, wenn der zweite Schritt der Duobinärkodierung, die Filterung durch das Filter 21 alleine realisiert ist. Dies kann beispielsweise der Fall sein, wenn die Übertragungseigenschaften der Sendeleiteranordnung sechs nicht bekannt sind, oder stark schwanken. Ebenso können weitere Filtermaßnahmen zur Unterdrückung von Störungen vorgesehen sein.

In einer besonders vorteilhaften Ausgestaltung der Erfindung weist die Sendeeinrichtung 8 noch eine zusätzliche Steuereinheit 25 des Senders auf. Mit dieser kann bevorzugt das Filter 21 gesteuert werden. Ein besonders großes Problem bei einer erfindungsgemäßen Anordnung ist die starke Schwankung der Übertragungsfunktion der Sendleiteranordnung 6. Diese wird nachfolgend durch die beiden Extremzustände beschrieben. Im ersten Fall befindet sich die Empfangskoppleranordnung 7 am Einspeisepunkt des Treibers 22 in die Sendeleiteranordnung 6. An dieser Stelle legt das eingespeiste Signal keinen oder nur einen vernachlässigbar geringen Weg in der Sendeleiteranordnung 6 zurück. Entsprechend ist auch die frequenzabhängige Dämpfung durch die Sendeleiteranordnung 6 vernachlässigbar. Es ist hier zur Erzielung einer optimalen Signalübertragung praktisch keine Frequenzgangskorrektur notwendig. Die andere Extremposition liegt an demjenigen Punkt, an dem die Empfangskoppleranordnung den größten Abstand zur Einkoppelstelle des Treibers in die Sendeleiteranordnung 6 aufweist. Dies ist häufig der Punkt, welcher am Drehübertrager exakt der Einkoppelposition gegenüberliegt. An dieser Stelle durchläuft das elektrische Signal den halben Umfang des Drehübertragers 3 und somit die halbe Leitungslänge der Sendleiteranordnung 6. Es ergibt sich hier eine sehr starke frequenzabhängige Dämpfung des Signals durch die Sendeleiteranordnung 6. Insbesondere im Falle der in der EP 1 012 899 A offenbarten Sendeleiteranordnung ergibt sich bei einer Signalauskopplung am Ort der Einspeisung nur eine sehr geringe Bandpasswirkung, da das Signal noch von keinem der mäanderförmigen Filterelemente durchlaufen wurde. Eine Signalauskopplung gegenüber dem Ort der Einspeisung wurde bereits eine größere Zahl der Filterelemente vom Signal durchlaufen. Entsprechend resultiert an der Stelle der Signal Auskopplung eine Bandpassfilterung hoher Ordnung. Entsprechend der Filterwirkung beziehungsweise der Position der Auskoppelstelle wird nun durch die Steuereinheit 25 des Senders das Filter 21 angesteuert, so dass es die Filterwirkung der Sendeleiteranordnung 6 zumindest weitgehend kompensiert und sich eine vorzugsweise konstante Übertragungsfunktion ergibt. Alternativ bzw. zusätzlich hierzu könnte anstelle der Steuerung eines Filters auch eine Steuerung des Treibers 22 erfolgen. Die Steuerung durch die Steuereinheit 25 des Senders erfolgt beispielsweise positionsabhängig. So ist die Dämpfungscharakteristik der Sendeleiteranordnung bekannt und kann beispielsweise bei Inbetriebnahme der gesamten Anordnung vermessen werden. So kann ein positionsabhängiger Korrekturwert, der entweder aus einem Messwertspeicher, oder auch durch Berechnung erzeugt wird, dem Filter 21 vorgegeben werden. Die Ermittlung der Position, d.h. der Weglänge zwischen der Empfangskoppleranordnung 7 und dem Einspeisepunkt der Signale des Treibers 22 in die Sendeleiteranordnung 6 kann durch einen Positionsenkoder oder auch durch eine Zeitmessung bei konstanter bzw. bekannter Drehgeschwindigkeit ermittelt werden. Eine weitere vorteilhafte Ausgestaltung der Erfindung umfasst einen Empfänger, welcher eine Steuereinheit 26 des Empfängers aufweist. Diese Steuereinheit optimiert die Schwellwerteinstellung des Detektors im Empfänger und kann diesen zusätzlich zu einer frequenzabhängigen Korrektur ansteuern. Hierzu erhält die Steuereinheit 26 Signale aus dem Verstärker 23. In einem einfachen Fall könnte beispielsweise die Steuereinheit 26 eine Vorrichtung zur Bestimmung der Signalamplitude des Signals aus dem Verstärker 23 oder eine Auswertung des Augenmusters vornehmen. Entsprechend könnten dann die Schaltschwellen des Dekoders, welcher die duobinären Signale dekodiert im Empfänger 24 angepasst werden. Alternativ hierzu könnte auch ein Verstärkungsfaktor im Empfänger 24 entsprechend eingestellt werden, so dass dieser mit fest vorgegebenen Schaltschwellen die Signale mit geringstmöglichen Fehlern dekodieren kann. Weiterhin könnte durch die Steuereinheit 26 auch eine zusätzliche Frequenzgangskompensation im Empfänger vorgenommen werden. So kann die Steuereinheit vorteilhafterweise die spektrale Zusammensetzung des Signals ermitteln und daraus einen Kompensationswert, insbesondere zur Anhebung hochfrequenter Signalanteile an den Empfänger 24 signalisieren. Zusätzlich bzw. alternativ zur spektralen Auswertung kann auch eine Auswertung der relativen Position zwischen dem Einspeisepunkt des Treibers 23 in die Sendeleiteranordnung 6 und der Empfangskoppleranordnung 7 herangezogen werden. Diese kann auf ähnlichem Wege, wie bereits zuvor bei der Steuereinheit 25 im Sender beschrieben, erfolgen.

In den Darstellungen zur Übertragungsfunktion beziehungsweise Dämpfung wird aus Gründen der Anschaulichkeit auf die Übertragungsfunktion der Sendeleiteranordnung 6 Bezug genommen, da diese, wie beispielsweise in der EP 1 012 899 A offenbart, den größten Einfluss auf die gesamte Übertragungsfunktion des Drehübertragers 3 hat. Selbstverständlich wird die Übertragungsfunktion des Drehübertragers 3 durch alle seine Komponenten, insbesondere die Sendeleiteranordnung 6 und die Empfangskoppleranordnung 7 bestimmt.

In einer weiteren vorteilhaften Ausgestaltung der Erfindung wird anstelle der duobinären Kodierung eine mb810 Kodierung oder ein anderer Leitungscode eingesetzt, beispielsweise 8b13bRLL(3,15). Die mb810 Kodierung bietet ein Ausganggsignal mit einem unter EMVaspekten günstigen Frequenzspektrum. Entsprechend ist dann nach der die Kodierung wieder eine entsprechende Verknüpfung mit dem Pseudozufallssignal vorzusehen, um das Originalsignal wiederherzustellen. Alternativ zu den RLL Codes können auch 8b12b, 8b13b oder 8b16 Kodierungen eingesetzt werden. Besonders günstig ist es, wenn zuerst eine mb810 Kodierung der Daten und dann mit den derart kodierten Daten eine duobinäre Kodierung durchgeführt wird.

In einer weiteren vorteilhaften Ausgestaltung der Erfindung ist noch wenigstens ein Multiplexer vorgesehen, der wenigstens zwei Signale zu einem gemeinsamen duobinär kodierten Signal kombiniert. Vorteilhafterweise weist dieser Multiplexer zur Reduzierung des gesamten Jittets noch einem FIFO bzw. einem digitalen Pufferspeicher auf. Die hierhin gespeicherten Daten werden dann duobinär kodiert über den Drehübertrager übertragen.

Zusätzlich zur duobinären Kodierung kann vorher noch eine digitale Kodierung beispielsweise in einem 8b10b oder einem 64b66b Code erfolgen.

Zur Vereinfachung der Darstellung wird in diesem Dokument auf eine Übertragung von dem rotierenden Teil auf das feststehende Teil eines Computertomographen Bezug genommen. Selbstverständlich ist eine erfindungsgemäße Vorrichtung auch in umgekehrter Übertragungsrichtung einsetzbar. Ebenso kann eine erfindungsgemäße Vorrichtung auch in anderen Anwendungen zur Drehübertragung und ebenso zur linearen Übertragung zweier gegeneinander beweglichen Einheiten herangezogen werden.

Ein erfindungsgemäßes Verfahren zur Übertragung von Daten zwischen dem rotierenden Teil 1 und dem stationären Teil 2 eines Computertomographen mit einem Drehübertrager 3 zur Übertragung elektrischer Signale mit einer Sendeleiteranordnung 6 und einer Empfangskoppleranordnung 7 umfasst die folgenden Schritte
a) Umwandlung der Signale von einer Datenquelle 4 durch wenigstens einen Kodierer 20 in duobinär und/oder mb810 kodierte Signale,
b) Filterung der kodierten Signale durch wenigstens ein Filter 21 zur Anpassung an die Übertragungseigenschaften des Drehübertragers 3,
c) Anpassung der Signale des Filters 21 an die elektrischen Eigenschaften der Sendeleiteranordnung 6 und Einspeisung dieser Signale in die Sendeleiteranordnung 6 durch wenigstens einen Treiber 22,
d) Verstärkung der Signale der Empfangskoppleranordnung 7 durch wenigstens einen Verstärker 23,
e) Dekodierung und Aufbereitung der Signale des Verstärkers 23 zur Weiterleitung an eine Datensenke 5 durch wenigstens einen Empfänger.

Bevorzugterweise erfolgt hier die Filterung des Signals durch das Filter 21 derart, dass Übertragungsfunktion des Filters 21 zusammen mit der Übertragungsfunktion der Sendeleiteranordnung 6 der Filtercharakteristik entspricht, wie sie für die Duobinärkodierung notwendig ist.

Die Übertragungsrichtung entsprechend dem Anspruch 1 wurde vom Rotor zum Stator gewählt, da dies dem häufigsten Einsatzfall entspricht. Allerdings ist ebenso eine Übertragung in entgegengesetzter Richtung oder aber auch bidirektional möglich. Der Übersichtlichkeit halber wird in diesem Dokument nicht zwischen der Übertragung zwischen gegeneinander beweglichen Einheiten und einer feststehenden und dazu beweglichen Einheiten unterschieden, da dies nur eine Frage des Ortsbezugs ist und keinen Einfluss auf die Funktionsweise der Erfindung hat.

Eine Zusammenstellung der Kodierungsprinzipien bei optischen Übertragungsstrecken findet sich in Michel et al.:"Ein 16 Gbit/s Duobinary Precoder-Chip in SiGe-Technologie sowie Möglichkeiten innovativer Aufbau- und Montagetechniken"; Vortrag am 21.06.2007 EEEfCOM 2007, Ulm. Dieses sei hiermit durch Referenz mit aufgenommen.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt in allgemeiner Form schematisch einen Computertomographen.
Fig. 2 zeigt schematisch die Anordnung von Sende/Empfangseinrichtungen.
Fig. 3 zeigt das Blockschaltbild einer erfindungsgemä-βen Anordnung.

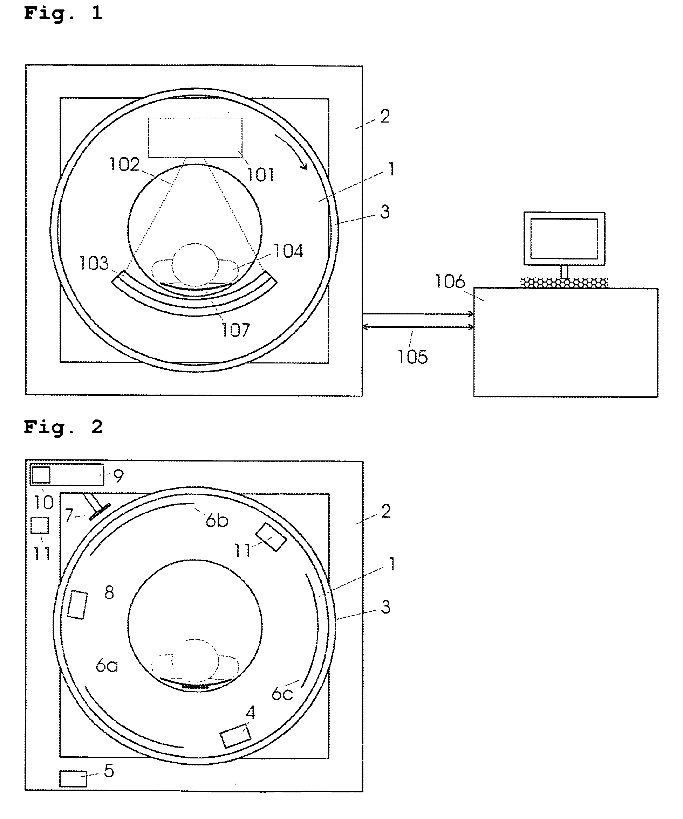

Fig. 1 zeigt eine erfindungsgemäße Vorrichtung am Beispiel eines Computertomographen. Der Computertomograph (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient 104 wird auf einer Liege 107 in der Öffnung des rotierenden Teils positioniert. Zur Abtastung des Patienten mittels Röntgenstrahlen 102 ist eine Röntgenröhre 101 sowie ein dieser gegenüberliegend angeordneter Detektor 103 vorgesehen. Röntgenröhre 101 und Detektor 103 sind auf dem rotierenden Teil 1 drehbar angeordnet. Ein Drehübertrager 3 dient zur elektrischen Verbindung zwischen dem rotierenden Teil 1 und dem stationären Teil 2. Hierbei werden einerseits die hohe elektrische Leistung zur Speisung der Röntgenröhre 101 in Richtung des rotierenden Teils 1 und gleichzeitig die Rohdaten des Bildes in der entgegengesetzten Richtung übertragen. Parallel hierzu ist eine Kommunikation von Steuerinformationen in beiden Richtungen vorgesehen. Eine Auswerte- und Steuereinheit 106 dient zur Bedienung des Computertomographen sowie zur Anzeige der erzeugten Bilder. Die Kommunikation mit dem Computertomographen erfolgt über eine bidirektionale Verbindung 105.

Fig. 2 zeigt in vereinfachte Form eine beispielhafte Anordnung eines erfindungsgemäßen Computertomographen mit zur Übertragung benötigten Komponenten. Die Daten einer Datenquelle 4 (Detektor 103 mit anschließender Signalverarbeitung bzw. DAS) an dem rotierenden Teil 1 werden mittels einer ersten Sendeeinrichtung 8 aufbereitet und an die Sendeleiteranordnung, welche hier beispielhaft aus drei Teilen 6a, 6b, 6c dargestellt ist, weitergeleitet. Diese Sendeleiteranordnung führt nun die hochfrequenten Signale. Diese werden von der Empfangskoppleranordnung 7 abgegriffen. Es ist beispielhaft eine Empfangskoppleranordnung, welche fest mit dem stationären Rahmen verbunden ist, dargestellt. Die von dieser Empfangskoppleranordnung 7 aufgefangenen Signale werden an eine erste Empfangseinrichtung 9 zur Aufbereitung weitergeleitet. Deren Ausgangsignale werden dann zu einer Datensenke 5 geführt.

In dieser Figur ist beispielhaft die PLL 10 in der Empfangseinrichtung 9 dargestellt. Ebenso könnte alternativ bzw. zusätzlich eine PLL in der Datensenke 5 vorhanden sein.

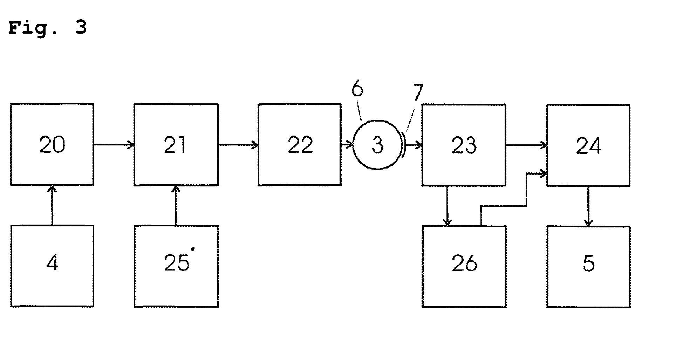

Fig. 3 zeigt das Blockschaltbild einer erfindungsgemä-βen Anordnung. Signale der Datenquelle 4 werden mittels eines Kodierers 20 in duobinäre Signale umgewandelt. Eine Anpassung an die Übertragungseigenschaften der Datenstrecke erfolgt mittels des Filters 21. Die Signale des Filters 21 werden dann mittels eines Treibers 22 in ihren Pegeln und der Impedanz an die elektrischen Eigenschaften der Sendeleiteranordnung 6 des Drehübertragers 3 angepasst und in diese Sendeleiteranordnung 6 eingekoppelt. Die Empfangskoppleranordnung 7 ist drehbar gegenüber der Sendeleiteranordnung 6 angeordnet und dient zum Abgriff der elektrischen Signale, welche in der Sendeleiteranordnung 6 geführt werden. Die von der Empfangskoppleranordnung 7 abgegriffenen elektrischen Signale werden nun über einen Verstärker 23 vorverstärkt und einem Empfänger 24 sowie optional einer Steuereinheit 25 zugeführt. Der Empfänger 24 dekodiert die duobinärkodierten Signale. Hierzu kann er vorteilhafterweise eine einstellbare Verstärkung, ein einstellbares Filter, insbesondere ein adaptives Filter, sowie einstellbare Schwellenwerte aufweisen. Eine Steuerung dieser Werte bzw. Filter kann mittels der Steuereinheit 26 in Abhängigkeit verschiedener Messgrö-βen erfolgen. Solche Messgrößen können beispielsweise die Signalamplitude des Eingangssignals aus dem Verstärker 23, das Augenmuster oder auch die relative Position zwischen der Empfangskoppleranordnung 7 und dem Einspeisepunkt 22 des Treibers in die Sendeleiteranordnung 6 sein. Das ausgewertete Signal des Empfängers 24 wird dann der Datensenke 5 zur weiteren Verarbeitung zugeführt. Um die Übertragungseigenschaften der gesamten Anordnung zu verbessern ist optional auf der Seite des rotierenden Teils 1 noch eine Steuereinheit 25 des Senders vorgesehen. Diese Steuereinheit steuert bevorzugt das Filter 21, optional aber auch andere Komponenten am rotierenden Teil. Besonders vorteilhaft ist es, wenn eine Steuerung des Filters 21 zur Anhebung hochfrequenter Signalanteile in Abhängigkeit von der Dämpfung der Sendeleiteranordnung 6 bzw. in Abhängigkeit von der Position zwischen der Empfangskoppleranordnung und dem Einspeisepunkt des Treibers 22 in die Sendeleiteranordnung 6 erfolgt.

### Bezugszeichenliste

- 1: Rotierendes Teil
- 2: Stationäres Teil
- 3: Drehübertrager
- 4: Datenquelle
- 5: Datensenke
- 6: Sendeleiteranordnung
- 7: Empfangskoppleranordnung
- 8: Sendeeinrichtung
- 9: Empfangseinrichtung
- 10: PLL
- 11: Steuereinheit
- 20: Digitaler Kodierer
- 21: Filter
- 22: Treiber
- 23: Verstärker
- 24: Empfänger
- 25: Steuereinheit Sender
- 26: Steuereinheit Empfänger
- 101: Röntgenröhre
- 102: Röntgenstrahlung
- 103: Detektor
- 104: Patient
- 105: bidirektionale Verbindung
- 106: Auswerte- und Steuereinheit
- 107: Patientenliege

## Patentansprüche

1. Computertomograph umfassend ein rotierendes Teil (1) sowie ein stationäres Teil (2), wobei zur Übertragung elektrischer Signale zwischen dem rotierenden Teil (1) und dem stationären Teil (2) ein Drehübertrager (3) mit einer Sendeleiteranordnung (6) umfassend mechanische Schleifringe oder kontaktlose elektrische Koppelelemente und einer Empfangskoppleranordnung (7) vorgesehen ist, umfassend
■ wenigstens eine Datenquelle (4),
■ wenigstens einen digitalen Kodierer (20) zur Umwandlung der Signale von der Datenquelle (4) in duobinär kodierte Signale,
■ wenigstens ein Filter (21) zur Anpassung der kodierten Signale an die Übertragungseigenschaften des Drehübertragers (3), derart, dass die Übertragungsfunktion des Drehübertragers (3) in Verbindung mit dem wenigstens einen Filter (21) ein zur Duobinärkodierung notwendiges Bandpassfilter darstellt,
■ wenigstens einen Treiber (22), welcher die Signale des Filters (21) an die elektrischen Eigenschaften der Sendeleiteranordnung (6) anpasst und diese in die Sendeleiteranordnung (6) einspeist,
■ wenigstens einen Verstärker (23), welcher die Signale der Empfangskoppleranordnung (7) verstärkt,
■ wenigstens einen Empfänger (24), welcher die Signale des Verstärkers (23) dekodiert und zur Weiterleitung an eine Datensenke (5) aufbereitet.

2. Drehübertrager (3) zur Übertragung elektrischer Signale zwischen einem rotierenden Teil (1) und einem stationären Teil (2) mit einer Sendeleiteran-ordnung (6) umfassend mechanische Schleifringe oder kontaktlose elektrische Koppelelemente und einer Empfangskoppleranordnung (7), umfassend
■ wenigstens einen digitalen Kodierer (20) zur Umwandlung der Signale von einer Datenquelle (4) in duobinär kodierte Signale,
■ wenigstens ein Filter (21) zur Anpassung der kodierten Signale an die Übertragungseigenschaften des Drehübertragers (3), derart, dass die Übertragungsfunktion des Drehübertragers (3) in Verbindung mit dem wenigstens einen Filter (21) ein zur Duobinärkodierung notwendiges Bandpassfilter darstellt,
■ wenigstens einen Treiber (22), welcher die Signale des Filters (21) an die elektrischen Eigenschaften der Sendeleiteranordnung (6) anpasst und diese in die Sendeleiteranordnung (6) einspeist,
■ wenigstens einen Verstärker (23), welcher die Signale der Empfangskoppleranordnung (7) verstärkt,
■ wenigstens einen Empfänger (24), welche die Signale des Verstärkers (23) dekodiert und zur Weiterleitung an eine Datensenke (5) aufbereitet.

3. Computertomograph bzw. Drehübertrager nach einem der vorhergehenden Ansprüche,
**dadurch gekennzeichnet dass**,
der wenigstens eine Kodierer (20) zuerst die Signale von der Datenquelle in mb810 oder run length limited (RLL) kodierte Signale wie 8b13bRLL(2,15) und diese dann anschließend in duobinär kodierte Signale umsetzt.

4. Computertomograph bzw. Drehübertrager nach einem der vorhergehenden Ansprüche,
**dadurch gekennzeichnet dass**,
eine Steuereinheit (25) des Senders vorgesehen ist, welche wenigstens das Filter (21) derart ansteuert, dass eine Anpassung an die Übertragungseigenschaften, insbesondere den Frequenzgang des Drehübertragers (3) erfolgt.

5. Computertomograph bzw. Drehübertrager nach einem der vorhergehenden Ansprüche,
**dadurch gekennzeichnet dass**,
die Steuereinheit (25) des Senders, wenigstens das Filter (21) derart ansteuert, dass eine Anhebung höherer Frequenzen erfolgt.

6. Computertomograph bzw. Drehübertrager nach einem der Ansprüche 4 oder 5,
**dadurch gekennzeichnet dass**,
die Steuereinheit (25) des Senders, wenigstens das Filter (21) abhängig von der Position der Empfangskoppleranordnung (7) gegenüber dem Einspeisepunkt des Treibers 22 in die Sendeleiteranordnung (6) ansteuert.

7. Computertomograph bzw. Drehübertrager nach einem der Ansprüche 4 oder 5,
**dadurch gekennzeichnet dass**,
die Steuereinheit (25) des Senders, wenigstens das Filter (21) abhängig der Signaldämpfung im Übertragungsweg des Drehübertragers (3) ansteuert.

8. Computertomograph bzw. Drehübertrager nach einem der vorhergehenden Ansprüche,
**dadurch gekennzeichnet dass**,
eine Steuereinheit (26) des Empfängers vorgesehen ist, welche wenigstens den Empfänger (24) derart ansteuert, dass eine Anpassung an die Übertragungseigenschaften, insbesondere den Frequenzgang des Drehübertragers (3) erfolgt.

9. Computertomograph bzw. Drehübertrager nach Anspruch 8,
**dadurch gekennzeichnet dass**,
die Steuereinheit (26) des Empfängers dem Empfänger (24) wenigstens eine Schaltstelle und/oder einen Verstärkungswert vorgibt.

10. Computertomograph bzw. Drehübertrager nach Anspruch 9,
**dadurch gekennzeichnet dass**,
die Steuereinheit (26) des Empfängers die Schaltschwelle und/oder den Verstärkungswert abhängig von der Position der Empfangskoppleranordnung (7) gegenüber dem Einspeisepunkt des Treibers 22 in die Sendeleiteranordnung (6) vorgibt.

11. Computertomograph bzw. Drehübertrager nach Anspruch 9,
**dadurch gekennzeichnet dass**,
die Steuereinheit (26) des Empfängers die Schaltschwelle und/oder denen Verstärkungswert abhängig von der Dämpfung des Drehübertragers oder der Amplitude des Eingangs Signals von der Empfangskoppleranordnung (7) vorgibt.

12. Signalübertragungseinrichtung für Computertomographen umfassend einen Drehübertrager nach einem der vorhergehenden Ansprüche.

13. Verfahren zur Übertragung von Daten zwischen dem rotierenden Teil (1) und dem stationären Teil (2) eines Computertomographen mit einem Drehübertrager (3) zur Übertragung elektrischer Signale mit einer Sendeleiteranordnung (6) umfassend mechanische Schleifringe oder kontaktlose elektrische Koppelelemente und einer Empfangskoppleranordnung (7) umfassend die folgenden Schritte:
a) Umwandlung der Signale von einer Datenquelle (4) durch wenigstens einen Kodierer (20) in duobinär kodierte Signale, wobei das für die duobinärkodierung notwendige Bandpassfilter aus einem Filter (21) und der Sendeleiteranordnung (6) gebildet wird,
b) Filterung der kodierten Signale durch wenigstens ein Filter (21) zur Anpassung an die Übertragungseigenschaften des Drehübertragers (3),
c) Anpassung der Signale des Filters (21) an die elektrischen Eigenschaften der Sendeleiteranordnung (6) und Einspeisung dieser Signale in die Sendeleiteranordnung (6) durch wenigstens einen Treiber (22),
d) Verstärkung der Signale der Empfangskoppleranordnung (7) durch wenigstens einen Verstärker (23),
e) Dekodierung und Aufbereitung der Signale des Verstärkers (23) zur Weiterleitung an eine Datensenke (5) durch wenigstens einen Empfänger.

## Claims

1. Computer tomograph comprising a rotating part (1) and also a stationary part (2), in which for transmission of electrical signals between the rotating part (1) and the stationary part (2) a rotary joint (3) with a transmission line arrangement (6) and a receiving coupler arrangement (7) are provided, comprising:
• at least one data source (4);
• at least one digital encoder (20) for converting the signals from the data source (4) to duobinary encoded signals;
• at least one filter (21) for matching the encoded signals to the transmission characteristics of the rotary joint (3), so that the transmission function of the rotary joint (3) together with the at least one filter (21) represents a band-pass filter needed for duobinary encoding;
• at least one driver (22) which matches the signals from the filter (21) to the electrical characteristics of the transmission line arrangement (6) and feeds these signals into the transmission line arrangement (6);
• at least one amplifier (23) which amplifies the signals from the receiving coupler arrangement (7);
• at least one receiver (24) which decodes the signals from the amplifier (23) and conditions these signals for relay to a data sink (5).

2. Rotary joint (3) for transmitting electrical signals between a rotating part (1) and a stationary part (2), with a transmission line arrangement (6) comprising mechanical sliprings or contactless electrical coupling elements and a receiving coupler arrangement (7), comprising:
• at least one digital encoder (20) for converting the signals from a data source (4) to duobinary encoded signals;
• at least one filter (21) for matching the encoded signals to the transmission characteristics of the rotary joint (3) so that the transmission function of the rotary joint (3) together with the at least one filter (21) represents a band-pass filter needed for duobinary encoding;
• at least one driver (22) which matches the signals from the filter (21) to the electrical characteristics of the transmission line arrangement (6) and feeds these signals into the transmission line arrangement (6);
• at least one amplifier (23) which amplifies the signals from the receiving coupler arrangement (7);
• at least one receiver (24) which decodes the signals from the amplifier (23) and conditions these signals for relay to a data sink (5).

3. Computer tomograph or rotary joint according to any one of the preceding claims,
**characterized in that**
the at least one encoder (20) first converts the signals from the data source to mb810 or run length limited (RLL) encoded signals such as 8b13bRLL(2,15), and then subsequently converts them to duobinary encoded signals.

4. Computer tomograph or rotary joint according to any one of the preceding claims,
**characterized in that**
a transmission control unit (25) is provided, which at least controls the filter (21) so that a matching to the transmission characteristics, in particular the frequency response, of the rotary joint is effected.

5. Computer tomograph or rotary joint according to any one of the preceding claims,
**characterized in that**
the transmission control unit (25) at least controls the filter (21) so that a preemphasis of higher frequencies is effected.

6. Computer tomograph or rotary joint according to any one of claims 6 or 7,
**characterized in that**
the transmission control unit (25) at least controls the filter (21) in dependence upon the position of the receiving coupler arrangement (7) with respect to the feeding-in point of the driver (22) into the transmission line arrangement (6).

7. Computer tomograph or rotary joint according to any one of claims 6 or 7,
**characterized in that**
the transmission control unit (25) at least controls the filter (21) in dependence upon the signal attenuation in the transmission path of the rotary joint (3).

8. Computer tomograph or rotary joint according to any one of the preceding claims,
**characterized in that**
a reception control unit (26) is provided, which at least controls the receiver (24) so that a matching to the transmission characteristics, and particularly to the frequency response of the rotary joint (3) is effected.

9. Computer tomograph or rotary joint according to claim 8,
**characterized in that**
the reception control unit (26) sets at least one switching threshold and/or an amplification value for the receiver.

10. Computer tomograph or rotary joint according to claim 9,
**characterized in that**
the reception control unit (26) sets the switching threshold and/or the amplification value in dependence upon the position of the receiving coupler arrangement (7) with respect to the point of feeding-in of the driver (22) into the transmission line arrangement (6).

11. Computer tomograph or rotary joint according to claim 9,
**characterized in that**
the reception control unit (26) sets the switching threshold and/or the amplification value in dependence upon the attenuation of the rotary joint or the amplitude of the input signal from the receiving coupler arrangement (7).

12. Signal transmission means for computer tomographs, comprising a rotary joint according to any one of the preceding claims.

13. Method for transmitting data between the rotating part (1) and the stationary part (2) of a computer tomograph having a rotary joint (3) for transmitting electrical signals with a transmission line arrangement (6) comprising mechanical sliprings or contactless electrical coupling elements and a receiving coupler arrangement (7), comprising the following steps:
a) converting the signals from a data source (4) with at least one encoder (20) to duobinary, and/or mb810 encoded signals, and/or run length limited (RLL) encoded signals such as 8b13bRLL(2,15);
b) filtering the encoded signals with at least one filter (21) for matching to the transmission characteristics of the rotary joint (3);
c) matching the signals from the filter (21) to the electrical characteristics of the transmission line arrangement (6), and feeding these signals into the transmission line arrangement (6) with at least one driver (22);
d) amplifying the signals from the receiving coupler arrangement (7) with at least one amplifier (23);
e) decoding and conditioning the signals from the amplifier (23) for relay to a data sink (5) with at least one receiver.

## Revendications

1. Appareil de tomodensitométrie comprenant une partie rotative (1) et une partie stationnaire (2), dans lequel est prévu pour la transmission de signaux électriques entre la partie rotative (1) et la partie stationnaire (2) un transmetteur rotatif (3) avec une disposition de conducteurs d'émission (6) comprenant des bagues frotteuses mécaniques ou des éléments de couplage électriques sans contact et une disposition de coupleurs de réception (7), comprenant
■ au moins une source de données (4),
■ au moins un codeur numérique (20) pour convertir les signaux de la source de données (4) en signaux à codage duobinaire,
■ au moins un filtre (21) pour adapter les signaux codés aux propriétés de transmission du transmetteur rotatif (3), de telle façon que la fonction de transmission du transmetteur rotatif (3) associée à l'au moins un filtre (21) représente un filtre passe-bande nécessaire au codage duobinaire,
■ au moins un excitateur (22) qui adapte les signaux du filtre (21) aux propriétés électriques de la disposition de conducteurs d'émission (6) et les fournit à la disposition de conducteurs d'émission (6),
■ au moins un amplificateur (23) qui amplifie les signaux de la disposition de coupleurs de réception (7),
■ au moins un récepteur (24) qui décode les signaux de l'amplificateur (23) et les prépare en vue de leur transmission à un collecteur de données (5).

2. Transmetteur rotatif (3) pour la transmission de signaux électriques entre une partie rotative (1) et une partie stationnaire (2) avec une disposition de conducteurs d'émission (6) comprenant des bagues frotteuses mécaniques ou des éléments de couplage électriques sans contact et une disposition de coupleurs de réception (7), comprenant
■ au moins un codeur numérique (20) pour convertir les signaux de la source de données (4) en signaux à codage duobinaire,
■ au moins un filtre (21) pour adapter les signaux codés aux propriétés de transmission du transmetteur rotatif (3), de telle façon que la fonction de transmission du transmetteur rotatif (3) associée à l'au moins un filtre (21) représente un filtre passe-bande nécessaire au codage duobinaire,
■ au moins un excitateur (22) qui adapte les signaux du filtre (21) aux propriétés électriques de la disposition de conducteurs d'émission (6) et les fournit à la disposition de conducteurs d'émission (6),
■ au moins un amplificateur (23) qui amplifie les signaux de la disposition de coupleurs de réception (7),
■ au moins un récepteur (24) qui décode les signaux de l'amplificateur (23) et les prépare en vue de leur transmission à un collecteur de données (5).

3. Appareil de tomodensitométrie ou transmetteur rotatif selon l'une des revendications précédentes, **caractérisé en ce que** l'au moins un codeur (20) convertit d'abord les signaux de la source de données en signaux codés en mb810 ou RLL (run length limited) tels que 8b13bRLL(2,15) et convertit ensuite ceux-ci en signaux à double codage binaire.

4. Appareil de tomodensitométrie ou transmetteur rotatif selon l'une des revendications précédentes, **caractérisé en ce qu'**il est prévu une unité de commande (25) de l'émetteur qui commande au moins le filtre (21) de façon à réaliser une adaptation aux propriétés de transmission, en particulier à la réponse en fréquence du transmetteur rotatif (3).

5. Appareil de tomodensitométrie ou transmetteur rotatif selon l'une des revendications précédentes, **caractérisé en ce que** l'unité de commande (25) de l'émetteur commande au moins le filtre (21) de façon à obtenir une élévation des fréquences les plus hautes.

6. Appareil de tomodensitométrie ou transmetteur rotatif selon l'une des revendications 4 ou 5, **caractérisé en ce que** l'unité de commande (25) de l'émetteur commande au moins le filtre (21) en fonction de la position de la disposition de coupleurs de réception (7) par rapport au point d'injection de l'excitateur (22) dans la disposition de conducteurs d'émission (6).

7. Appareil de tomodensitométrie ou transmetteur rotatif selon l'une des revendications 4 ou 5, **caractérisé en ce que** l'unité de commande (25) de l'émetteur commande au moins le filtre (21) en fonction de l'atténuation du signal dans le trajet de transmission du transmetteur rotatif (3).

8. Appareil de tomodensitométrie ou transmetteur rotatif selon l'une des revendications précédentes, **caractérisé en ce qu'**il est prévu une unité de commande (26) du récepteur qui commande au moins le récepteur (24) de façon à obtenir une adaptation aux propriétés de transmission, en particulier à la réponse en fréquence du transmetteur rotatif (3).

9. Appareil de tomodensitométrie ou transmetteur rotatif selon la revendication 8, **caractérisé en ce que** l'unité de commande (26) du récepteur fixe au récepteur (24) au moins un point de commutation et/ou une valeur d'amplification.

10. Appareil de tomodensitométrie ou transmetteur rotatif selon la revendication 9, **caractérisé en ce que** l'unité de commande (26) du récepteur fixe le seuil de commutation et/ou la valeur d'amplification en fonction de la position de la disposition de coupleurs de réception (7) par rapport au point d'injection de l'excitateur (22) dans la disposition de conducteurs d'émission (6).

11. Appareil de tomodensitométrie ou transmetteur rotatif selon la revendication 9, **caractérisé en ce que** l'unité de commande (26) du récepteur fixe le seuil de commutation et/ou la valeur d'amplification en fonction de l'atténuation du transmetteur rotatif ou de l'amplitude du signal d'entrée de la disposition de coupleurs de réception (7).

12. Dispositif de transmission de signaux pour appareils de tomodensitométrie comprenant un transmetteur rotatif selon l'une des revendications précédentes.

13. Procédé pour la transmission de données entre la partie rotative (1) et la partie stationnaire (2) d'un appareil de tomodensitométrie avec un transmetteur rotatif (3) pour la transmission de signaux électriques avec une disposition de conducteurs d'émission (6) comprenant des bagues frotteuses mécaniques ou des éléments de couplage électriques sans contact et une disposition de coupleurs de réception (7), comprenant les étapes suivantes :
a) conversion des signaux d'une source de données (4) en signaux à codage duobinaire par au moins un codeur (20), le filtre passe-bande nécessaire au codage duobinaire étant formé par un filtre (21) et la disposition de conducteurs d'émission (6),
b) filtrage des signaux codés par au moins un filtre (21) en vue de leur adaptation aux propriétés de transmission du transmetteur rotatif (3),
c) adaptation des signaux du filtre (21) aux propriétés électriques de la disposition de conducteurs d'émission (6) et injection de ces signaux dans la disposition de conducteurs d'émission (6) par au moins un excitateur (22),
d) amplification des signaux de la disposition de coupleurs de réception (7) par au moins un amplificateur (23),
e) décodage et préparation des signaux de l'amplificateur (23) en vue de leur transmission à un collecteur de données (5) par au moins un récepteur.
